# EUROPEAN PATENT APPLICATION

(11) **EP 1 745 808 A1**
(43) Date of publication of application: **24.01.2007**
(21) Application number: 05734508.4
(22) Date of filing: 19.04.2005
(51) Int. Cl.: A61M 1/02, A61M 1/34, A61M 1/36, B01D 39/16

(54) **MEDICAL FILTER MATERIAL AND, UTILIZING THE SAME, EXTRACORPOREAL CIRCULATION COLUMN AND BLOOD FILTER**

(30) Priority: 21.04.2004 JP 2004125510
(71) Applicant: TORAY INDUSTRIES, INC., Tokyo 103-8666 (JP)
(72) Inventor: NARUSE, Yoshihiro, 5200842 (JP); NONAKA, Shuichi;, Otsu-shi, Shiga; 5200842 (JP); OCHI, Takashi, 4110033 (JP)
(74) Representative: Kador & Partner
(86) International application number: PCT/JP2005/007426
(87) International publication number: WO 2005/102413

(57) **Abstract**

A medical filter material **characterized by** comprising a dispersion of nanofibers of thermoplastic polymer having a number average diameter of 1 to 500 nm wherein the ratio of single fibers with a diameter of more than 500 nm and 1 µm or less is 3% or less in terms of weight ratio. Further, there are provided, utilizing the medical filter material, an extracorporeal circulation column and a blood filter. Through the employment of nanofibers small in fiber diameter dispersion, high in strength and high in productivity, there can be provided a medical filter material excellent in hemadsorption performance and protein adsorption performance. Through packing with this medical filter material, there can be provided high-performance extracorporeal circulation column and blood filter.

## Description

### Technical Field of the Invention

The present invention relates to a medical filter material using nanofibers, and an extracorporeal circulation column and a blood filter utilizing the filter material. Where, the term of "nanofibers" in the present invention means single fibers having a number average diameter of the single fibers in a range of 1 nm or more and 1µm or less. Further, this nanofiber may be in a fibrous form, and it is not limited in length, sectional shape and the like. More specifically, the present invention relates to a medical filter material using such nanofibers, and in particular, relates to a medical filter material using novel nanofibers which can develop the use in the medical field based on the fineness, that has not been achieved in the conventional technology, and the uniformity of the fineness, and an extracorporeal circulation column and a blood filter utilizing the filter material.

### Background Art of the Invention

In inflammatory diseases such as ischaemic recirculation lesion of organs, septicemia, ulcerative colitis, clonal diseases, SIRS and infectious diseases, it has been known that inflammatory leukocytes such as granules increase in blood, and it is important to remove the inflammatory leukocytes by extracorporeal circulation. Further, recently, so-called leukocyte removal blood transfusion, in that a blood product is transfused after mixed leukocytes contained in the blood product are removed, is spreading. This is because it has been clarified that a relatively light side reaction such as a headache, a nausea, a chill or a non-hemolytic feverish reaction accompanying with blood transfusion, or a critical side reaction such as an alloantigen sensitization, a virus infection or GVHD after blood transfusion which gives a serious affection to a blood recipient, is caused mainly by leukocytes mixed in a blood product which has been used for blood transfusion.

From such circumstances, as a filter material for removing leukocytes, for example, a leukocyte removal filter, in which very thin cellulosic fibers are held onto a medical filter using PET (polyethylene terephthalate) melt-blow fibers having a diameter of several micron meters, is proposed (Patent document 1). Indeed, the leukocyte removal filter material described in Patent document 1 is excellent in ability for removing leukocytes, but, because it uses cellulose fibril and there occurs a dispersion in the diameter of cellulose fibers in the fibrillation, there is a fear that the leukocyte removal is not carried out uniformly in a local part of the filter material. Further, although a method for making acetic bacteria produce cellulose fibers having a diameter of several tens nanometer order is also proposed in Patent document 1 in order to unify the fiber diameter, the absolute strength of the fiber is low because the fiber is too thin, there are a problem that it becomes necessary to increase the pressure resistance for treating a large amount of blood, and a problem that the productivity of the filter material cannot be improved because it takes a long time to produce cellulose by acetic bacteria.

From this point of view, a medical filter material has been required, which utilizes nanofibers of a synthetic polymer small in dispersion of diameter of single fibers, high in absolute strength of fiber and high in productivity.

On the other hand, as a technology for obtaining a substrate of synthetic fibers of nanometer level that is paid attention to recently, a technology of electrospinning is proposed (Non-patent documents 1 and 2). This is a technology for dissolving a polymer in electrolytic solution and extruding it from a die, and at that time, applying a high voltage in a range of several thousands volt to 30,000 volt, and achieving a fine fibrillation by a highspeed jet of polymer solution and the successive bending and the expansion of the jet. When this technology is used, a nanofiber nonwoven fabric of nanofibers having a diameter of several hundreds nm can be obtained, and if the conditions of the polymer and the spinning are specified, there is a case where a substrate of nanofibers having a diameter corresponding to several tens nm can be obtained. However, because the nanofibers obtained by electrospinning are fibers obtained by evaporating solvent at the process of fibrillation, the nanofibers frequently are not oriented and crystallized, and only fibers low in strength have been obtained. Therefore, it has been difficult to obtain a nanofiber substrate high in absolute strength suitable as a medical filter material. Moreover, the electrospinning has big problems as a production method, that is, there are a problem that the size of an obtained medical filter material is about 100 cm² at largest, and a problem that the productivity is several grams/hour at highest and is much low as compared with that of a usual melt-spinning. Furthermore, because a high voltage is required and harmful organic solvent and very fine yarns are suspended in air, there is also a problem that electric shock, explosion or poisoning may occur.
Patent document 1: WO97/23266
Non-patent document 1: Polymer, vol.40, 4585-4592 (1999)
Non-patent document 2: Polymer, vol.43, 4403-4412 (2002)

### Disclosure of the Invention

### Problems to be solved by the Invention

As is evident from the above-described explanation, a medical filter material, which is not restricted in shape and polymer, which can be applied in the various fields, and which utilizes nanofibers uniform in diameter of single fiber, high in strength and high in productivity, has been required.

Accordingly, an object of the present invention is to provide a medical filter material using novel nanofibers excellent in hemadsorption performance and protein adsorption performance by utilizing nanofibers small in dispersion of fiber diameter, high in strength and high in productivity which have not been realized in the conventional technologies.

Further, another object of the present invention is to provide high-performance medical equipment and materials, particularly, an extracorporeal circulation column and a blood filter utilizing such a medical filter material.

### Means for solving the Problems

To achieve the above-described objects, a medical filter material according to the present invention is characterized by comprising a dispersion of nanofibers of thermoplastic polymer having a number average diameter of 1 nm or more and 500 nm or less wherein a ratio of single fibers with a diameter of more than 500 nm and 1 µm or less is 3% or less in terms of weight ratio.

Further, an extracorporeal circulation column and a blood filter according to the present invention are characterized in that such a medical filter material is packed therein.

### Effect according to the Invention

In the medical filter material according to the present invention, because nanofibers small in dispersion of fiber diameter, high in strength and high in productivity are used, a medical filter material excellent in hemadsorption performance and protein adsorption performance can be realized. By packing this medical filter material, high-performance medical equipment and materials, particularly, an extracorporeal circulation column and a blood filter can be provided.

### Brief explanation of the drawings

[Fig. 1] Fig. 1 is a diagram showing a result of observing a cross section of nanofibers in the present invention by TEM.
[Fig. 2] Fig. 2 is a diagram showing a result of observing an example of a net structure in the present invention by SEM.
[Fig. 3] Fig. 3 (A) is a schematic diagram of an extracorporeal circulation column according to an embodiment of the present invention, and Fig. 3 (B) is a schematic cross-sectional view of the column packed with a filter material in a form of a lattice.
[Fig. 4] Fig. 4 (A) is a schematic diagram of a blood filter according to an embodiment of the present invention and Fig. 4
   (B) is a schematic cross-sectional view of the blood filter packed with a filter material in a form of a lattice (B).
[Fig. 5] Fig. 5 is a schematic diagram of a spinning machine used in Examples.
[Fig. 6] Fig. 6 is a schematic vertical sectional view of a die used in Examples.
[Fig. 7] Fig. 7 is a schematic diagram of a drawing machine used in Examples.
[Fig. 8] Fig. 8 is a diagram showing a result of observing a cross section of polymer alloy fibers in Example 1 by TEM.
[Fig. 9] Fig. 9 is a graph indicating an example of dispersion in diameter of nanofibers relative to frequency.
[Fig. 10] Fig. 10 is a graph indicating an example of dispersion in diameter of nanofibers relative to fiber ratio.
[Fig. 11] Fig. 11 is a diagram showing a result of observing a surface of a filter material after evaluation of blood adsorption in Example 1 by SEM.
[Fig. 12] Fig. 12 is a diagram showing a result of observing a surface of a filter material after evaluation of blood adsorption in Comparative Example 1 by SEM.
[Fig. 13] Fig. 13 is a diagram showing a result of evaluation of protein adsorption by SDS-PAGE in Example 2 and Comparative Example 3.
[Fig. 14] Fig. 14 is a schematic vertical sectional view of an extruder used in Examples.
[Fig. 15] Fig. 15 is a diagram showing a result of observing a surface of a filter material having a net structure in Example 15 by SEM.
[Fig. 16] Fig. 16 (A) is a perspective view of a filter material according to an embodiment of the present invention, Fig. 16 (B) is a perspective view showing a packing form of the filter material into a column, and Fig. 16 (C) is a schematic vertical sectional view of the column the liquid flow of which is cross flow to the filter material.
[Fig. 17] Fig. 17 (A) is a partial perspective view of a filter material according to another embodiment of the present invention, and Fig. 17 (B) is a schematic vertical sectional view of a column the liquid flow of which is parallel flow to the filter material.

### Explanation of symbols

- 1:: extracorporeal circulation column
- 2:: blood introduction port
- 3:: blood discharge port
- 4:: filter material
- 5:: blood filter
- 6:: hopper
- 7:: chip supplying part
- 7a:: melting part
- 8:: spin block
- 9:: spinning pack
- 10:: die
- 11:: chimney
- 12:: yarn
- 13:: collecting and oiling guide
- 14:: first godet roller
- 15:: second godet roller
- 16:: wound yarn
- 17:: metering zone
- 18:: length of discharge hole
- 19:: diameter of discharge hole
- 20:: undrawn yarn
- 21:: feed roller
- 22:: first hot roller
- 23:: second hot roller
- 24:: third roller (room temperature)
- 25:: drawn yarn
- 26:: leukocyte
- 27:: erythrocyte
- 28:: extruder
- 29:: rod made of polymer alloy fibers
- 30:: piston
- 31:: discharge hole
- 32:: hole for liquid passage
- A:: polymer alloy fiber
- F:: nanofiber
- N:: nylon
- P:: polyester
- S:: substrate fiber

### The Best mode for carrying out the Invention

Hereinafter, a medical filter material utilizing nanofibers according to the present invention will be explained in more detail together with desirable embodiments.
A medical filter material utilizing nanofibers according to the present invention includes a dispersion of nanofibers of thermoplastic polymer having a number average diameter of 1 nm or more and 500 nm or less wherein a ratio of single fibers with a diameter of more than 500 nm and 1µm or less is 3% or less in terms of weight ratio.

In the present invention, these two factors are particularly important: one is that the number average diameter of nanofibers is small, and the other is that fibers with a diameter of more than 500 nm and 1 µm or less is 3% or less in terms of weight ratio, namely, fibers with a large diameter almost do not exist except nanofibers having a number average diameter of 1 nm or more and 500 nm or less.

Where, the term of "nanofibers" in the present invention means single fibers having a fiber diameter in a range of 1 nm or more and 1 µm or less, and the term of "dispersion of nanofibers" means a material having a form dispersed with the nanofibers. Further, the nanofiber may be in a fibrous form, and it is not limited in length, shape of cross section and the like.

In the present invention, the number average diameter of nanofibers is determined by observing a cross section or surface of a medical filter material by a transmission electron microscope (TEM) or a scanning electron microscope (SEM), measuring diameters of 50 or more fibers sampled randomly in an identical cross section, carrying out this measurement at three or more positions, and measuring diameters of at least totally 150 or more fibers.

In this case, fibers with a diameter more than 1µm are not counted for the determination of diameter of nanofibers. Further, in a case where a nanofiber has a deformed section (non-circular section), first, the cross-sectional area of the nanofiber is measured, and the area is assumed to be the area of the cross section of circular one. By calculating a diameter from such an area, the number average diameter of the nanofibers having the deformed section can be determined. Here, the average value of the number average diameter of the nanofibers is determined by measuring the diameters of nanofibers down to 0.1 nm, rounding the measured value to unit, and determining a simple average value.

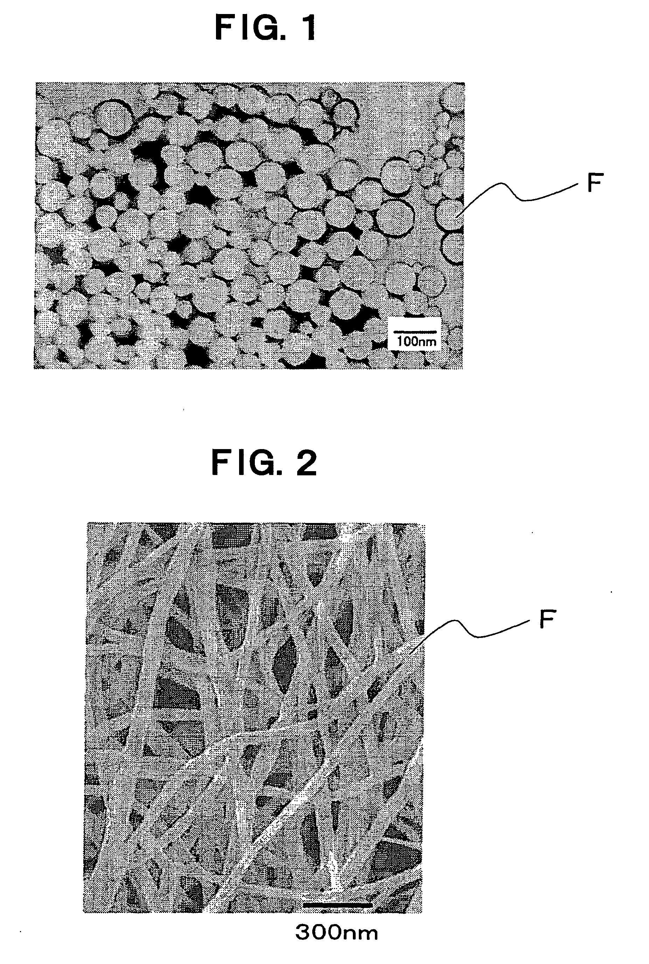

Where, in order to explain an example of a structure of a medical filter material using the nanofibers according to the present invention, a result of observing a cross section of nanofibers used in a medical filter material in the present invention by a microscope is shown in Fig. 1. The label F in Fig. 1 indicates nanofibers. As shown in Fig. 1, in the nanofibers forming the medical filter material according to the present invention, while almost all of them have a nanofiber diameter of 500 nm or less, the diameter of the nanofibers indicates a distribution from about 10 nm to about 100 nm.

If the number average diameter of nanofibers is 1 nm or more, because the absolute strength as a fiber can be ensured to some extent, for example, it can be suppressed that the fibers are likely to be cut by hemocyte component or other coarse components, and the reliability of the filter can be increased. Further, if the number average diameter is 500 nm or less, a surface area enough to adsorb hemocyte or protein can be ensured, and a selective adsorption performance ascribed to nanofiber structure exhibits and the performance of the filter can be improved. From the viewpoint of increasing the absolute strength of nanofibers, the number average diameter of nanofibers is preferably greater, and it is preferably 30 nm or more. On the other hand, from the viewpoint of improving the performance of the filter, the number average diameter of nanofibers is preferably smaller, and it is preferably 200 nm or less, more preferably 80 nm or less.

Further, in the present invention, the fiber ratio of single fibers with a diameter of more than 500 nm and 1µm or less in the medical filter material means a weight ratio of thick fibers (single fibers with a diameter of more than 500 nm and 1µm or less) relative to the weight of the whole of nanofibers, and it is calculated as follows. Namely, respective diameters of single fibers of nanofibers in the medical filter material are referred to as "di", and the total sum of squares thereof is calculated ((d₁²+d₂²+ ··· +dₙ²) = Σdᵢ²(i=1-n)). Further, respective diameters of nanofibers in a range of more than 500 nm and 1µm or less are referred to as "Di", and the total sum of squares thereof is calculated ((D)₁²+D₂²+ ··· +Dₘ²) = ΣDᵢ²(i=1-m)). By calculating the rate of ΣDᵢ² to Σdᵢ², the area ratio of fibers with a diameter in a range of more than 500 nm and 1µm or less relative to all nanofibers, that is, the ratio in terms of weight ratio, can be determined.

In the medical filter material using nanofibers according to the present invention, it is important that the fiber ratio of single fibers with a diameter in a range of more than 500 nm and 1µm or less is 3% or less in terms of weight ratio, more preferably 1% or less, and further preferably 0.1% or less. Namely, this means that the presence of thick nanofibers with a diameter more than 500 nm is nearly zero. Further, in a case where the number average diameter of nanofibers is 200 nm or less, the fiber ratio of single fibers with a diameter more than 200 nm is preferably 3% or less, more preferably 1% or less, and further preferably 0.1% or less. Further, in a case where the number average diameter of nanofibers is 100 nm or less, the fiber ratio of single fibers with a diameter more than 100 nm is preferably 3% or less, more preferably 1% or less, and further preferably 0.1 % or less. By these, the function of the medical filter material using the nanofibers can be sufficiently exhibited, and the stability in quality of a product will be good.

In the present invention, it is important that the nanofibers are made of a thermoplastic polymer. In the nanofibers made of a thermoplastic polymer, the fiber diameter can be controlled to be much uniform as compared with the beating of cellulose fibril, besides, the strength thereof can be made to be much higher than that of semi-synthetic fibers such as natural fibers or a rayon, and further, because they can obtained by melt-spinning, the productivity is very high and it becomes possible to easily obtain the nanofibers.

Although polyester, polyamide, polyolefin, polyphenylene sulfide (PPS), etc. can be exemplified as the thermoplastic polymer in the present invention, condensation polymerization-group polymers represented by polyester and polyamide have a high melting point in most cases, and therefore, they are more preferable. If the melting point of the polymer is 165°C or higher, the thermal resistance of nanofibers is good, and such a condition is preferable. For example, the melting point is 170°C in polylactic acid (PLA), 255°C in PET, and 220°C in N6 (nylon 6).

The medical filter material according to the present invention is used, for example, as a substrate for treating a body fluid. The body fluid expressed here means blood, plasma, serum, ascites, lymph, articular axil and fraction obtained therefrom, other liquid components ascribed to organ, etc., and the medical filter material according to the present invention is suitably used for removing unnecessary leukocytes, toxine, protein, etc. by filtering or adsorbing components in a body fluid.

Further, the form of the medical filter material according to the present invention is not particularly limited, and nanofibers may be contained as at least a part. A preferable form of the medical filter material has a large surface area in order to the efficiency of filtration or adsorption, and a woven fabric, a knit fabric, a nonwoven fabric, a paper, a film, a composite thereof, etc. are particularly preferred.

Further, although the content of nanofibers in the medical filter material according to the present invention is not particularly limited, the content is preferably 0.0001% by weight or more relative to the filter material, more preferably 0.01% by weight or more.

The nanofibers in the present invention preferably form, for example, a net structure. Where, the net structure means a state where nanofibers are not formed as a bundle and respective nanofibers are in an opened condition so that single fibers are dispersed to form a state with pores, and a plurality of single fibers are physically or chemically entangled to form a net structure. As an example of the net structure, a result of observing a form dispersed with nanofibers by an electron microscope is shown in Fig. 2. In Fig. 2, label F indicates nanofibers. The nanofibers are dispersed at a single fiber level by the net structure formed by the nanofibers, and the surfaces of the nanofibers, namely, the adsorption sight, can be utilized effectively. By that, components in a body fluid required to be removed can be trapped efficiently.

The filter material according to the present invention is preferably made of only nanofibers in order to increase the adsorption efficiency of hemocytes or protein relative to the weight of the filter material. By the structure of the filter material made by only nanofibers, the large surface area of the nanofibers can be utilized as much as possible, and the filtration or adsorption efficiency can be sought up to the limit. Further, by the condition where substances other than nanofibers are not contained in the filter material, the trapping of components in a body fluid required to be removed can be carried out uniformly.

Further, in a case where a liquid flow perpendicular to the medical filter material is formed, the medical filter material according to the present invention is preferably formed from nanofibers and a fiber substrate having a number average diameter of more than 1 µm and 100µm or less. Where, the fiber substrate means a substrate having a function as a supporting material for nanofibers, it may be a fibrous substrate, and as the fiber substrate, a woven fabric, a knit fabric, a nonwoven fabric, a paper, etc. can be exemplified. By the existence of other fibers with a diameter greater than that of nanofibers in the medical filter material, exhibition of an advantage, that has been achieved by only nanofibers, can be expected. For example, the strength is small by only nanofibers and it cannot bear a practical use, but, by using the nanofibers together with the fiber substrate having a diameter greater than that the nanofibers, while the adsorption effect and the like due to the large surface area of nanofibers is exhibited, the mechanical strength as a structural material can be increased, and an effect of reinforcement can be obtained.

Further, in a case where nanofibers having a high hydrophilicity are used, when a body fluid is treated, although a dimensional change may happen by water adsorption swelling and the like, the dimensional stability can be increased by using the above-described fiber substrate together.

Further, in a case where the medical filter material is formed by only nanofibers, the gap created between a plurality of nanofibers (opened seam) becomes extremely small, usually thereby increasing the pressure loss and reducing the degree of passing a body fluid, but, by using the fiber substrate together, the gap created between fibers with a diameter greater than that of nanofibers becomes large, as a result the apparent density becomes small, and a low-pressure loss, which is a required property in use of a medical filter for removing unnecessary substances in a body fluid by filtration system, can be achieved. The number average diameter of the fiber substrate is preferably in a range of 2 to 50µm, more preferably in a range of 3 to 20µm.

It is preferred that the weight of the medical filter material according to the present invention is in a range of 1 to 500 g/m². If the weight is in a range of 1 to 500 g/m², not only a sufficient strength can be given to the filter material, but also the easiness to be bent and the workability increase, and for example, there is such a merit as the filter material is liable to be easily packed into an extracorporeal circulation column or a blood filter. The weight is more preferably in a range of 1 to 350 g/m², and further preferably in a range of 5 to 280 g/m².

It is preferred that the apparent density of the medical filter material according to the present invention is in a range of 0.01 to 1.0 g/cm³. If the apparent density is in a range of 0.01 to 1.0 g/cm³, the pressure loss can be controlled small as well as a sufficient strength can be given to the filter material. The apparent density is more preferably in a range of 0.05 to 0.4 g/cm³, and further preferably in a range of 0.07 to 0.3 g/cm³.

In the medical filter material according to the present invention, depending upon the substance required to be filtered or adsorbed, the surface of the filter material can be modified. As a method for modifying the surface of the filter material, a surface graft polymerization, coating of polymer material or treatment in a bath of a polymer material solution or dispersion, electric discharge treatment, etc. can be exemplified. Although the polymer material used for the modification of the filter material by surface graft polymerization or coating of polymer material or treatment in a bath of a polymer material solution or dispersion is not particularly limited, from the purpose of giving a hydrophilicity, a polymer material having a non-ion hydrophilic group is preferable. As the non-ion hydrophilic group, hydroxyl group, amide group, polyethylene oxide chain, etc. can be exemplified. As a monomer capable of being used for synthesis of a polymer material having a non-ion hydrophilic group, for example, 2-hydroxyethyl methacrylate, 2-hydroxyethyl acrylate, vinyl alcohol (one prepared by hydrolizing a polymer prepared by polymerization of vinyl acetate), methacrylamide, N-vinyl pyrolidone, etc. can be exemplified. Among the above-described monomers, 2-hydroxyethyl methacrylate and 2-hydroxyethyl acrylate are preferred.

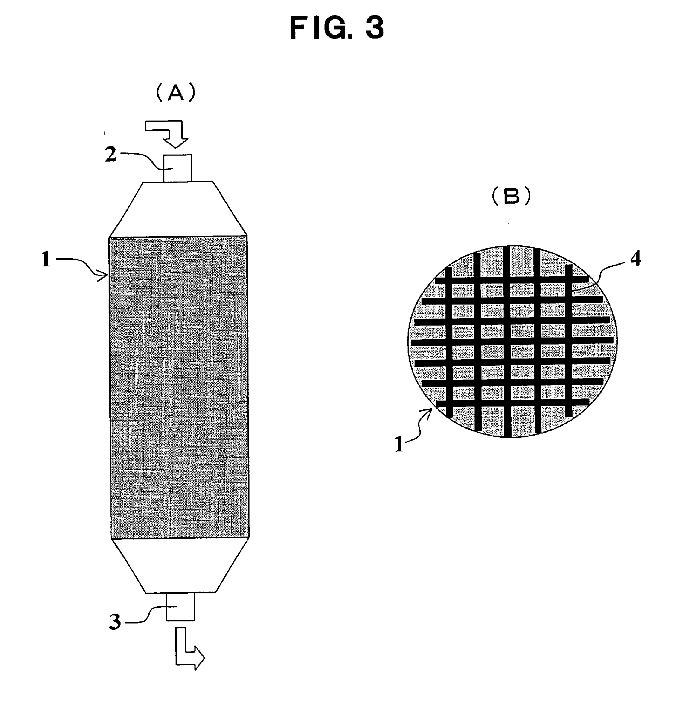

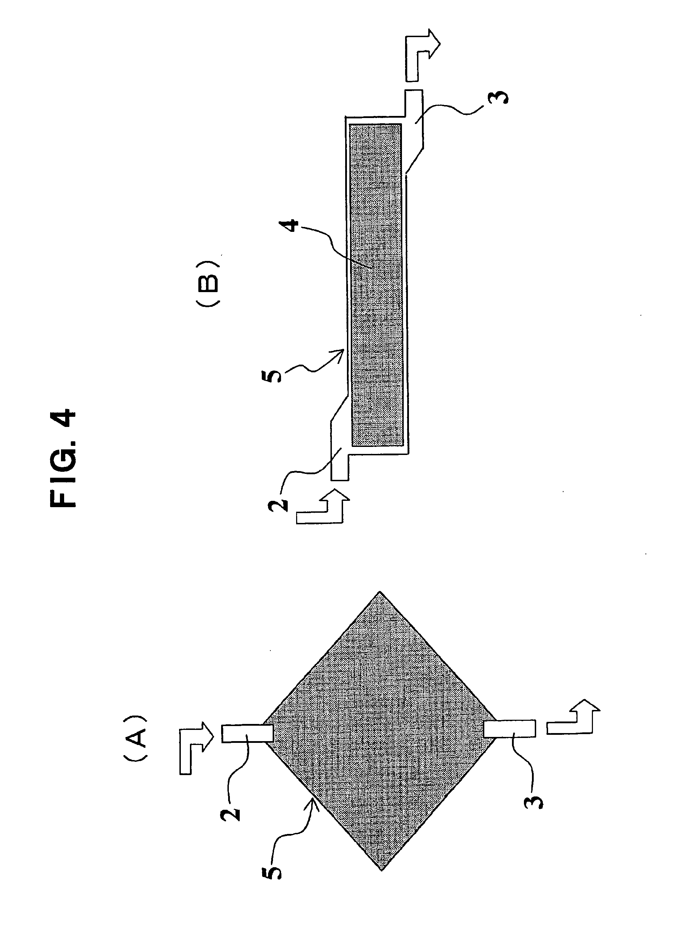

The medical filter material according to the present invention can be formed as an extracorporeal circulation column or a blood filter by being packed in a vessel having a body fluid introduction port at its upper part and a body fluid discharge port at its lower part. A structural example of an extracorporeal circulation column is shown in Fig. 3, and a structural example of a blood filter is shown in Fig. 4, respectively.

In Fig. 3 (A), label 1 indicates an extracorporeal circulation column, label 2 indicates a blood introduction port and label 3 indicates a blood discharge port, and in this extracorporeal circulation column 1, a filter material 4 according to the present invention is packed as shown in Fig. 3 (B). In Fig. 4 (A), label 5 indicates a blood filter, label 2 indicates a blood introduction port and label 3 indicates a blood discharge port, and in this blood filter 5, the filter material 4 according to the present invention is packed as shown in Fig. 4 (B).

Further, in the extracorporeal circulation column or the blood filter according to the present invention, it is preferred that the filter material is packed so as to form a liquid flow of cross flow or parallel flow to the filter material. In a case where the liquid flow is perpendicular to the filter material, by filtering a body fluid, it becomes possible to remove relatively large unnecessary components such as hemocytes efficiently. On the other hand, in a case where the liquid flow is parallel to the filter material, it becomes possible to remove protein, toxine, etc. in a body fluid by adsorption.

In the cross flow, because there is a case where coarse components such as hemocytes clog when the apparent density of the filter material is high, in such a case, the parallel flow may be employed. Further, in the parallel flow, in a case where components required to be removed are contained in a body fluid, if the adsorption sight of the surface of the filter material is covered, the adsorption cannot be carried out any more, in such a case, the cross flow may be employed. With respect to packing of filter material, any one flow style may be employed depending upon the purpose of removal or the form of the filter material, and further, it is possible to make an extracorporeal circulation column or a blood filter combining the parallel flow and the perpendicular flow.

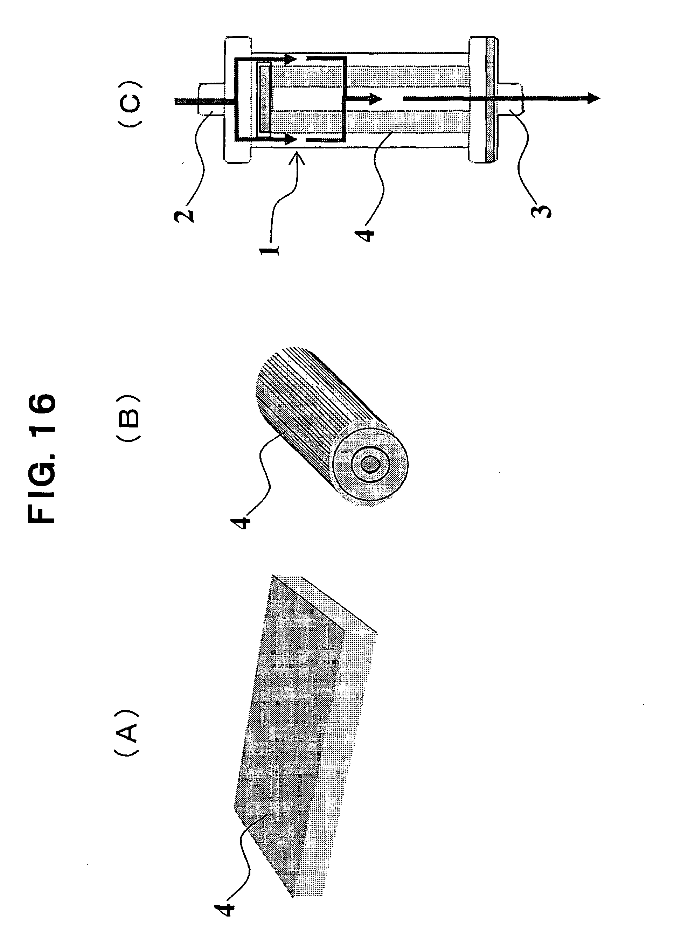

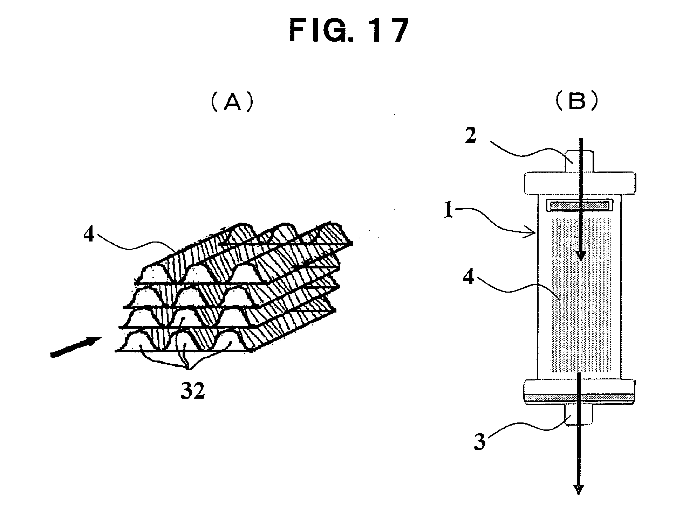

A structural example of a column in a case of perpendicular flow relative to the filter material is shown in Fig. 16, and a structural example of a column in a case of parallel flow relative to the filter material is shown in Fig. 17, respectively.

In Fig. 16, label 1 indicates an extracorporeal circulation column, label 2 indicates a blood introduction port, label 3 indicates a blood discharge port, and label 4 indicates a filter material, respectively, and in this extracorporeal circulation column 1, the filter material shown in Fig. 16 (A) is packed after being wound as shown in Fig. 16 (B). The perpendicular flow is formed, for example, so that the blood flows from the outside toward the inside relative to the wound filter material 4 as shown by arrows in Fig. 16 (C). Where, as to the blood flow direction, the extracorporeal circulation column can also be designed so that the blood flows from the inside toward the outside relative to the filter material 4 in accordance with the purpose. Further, in Fig. 17, label 1 indicates an extracorporeal circulation column, label 2 indicates a blood introduction port, label 3 indicates a blood discharge port, label 4 indicates a filter material, and label 32 indicates holes for liquid passage extending in the blood flow direction shown by the arrow, respectively. In the extracorporeal circulation column 1, the filter material 4 is packed after processing it as a three-dimensional filtration structural material shown in Fig. 17 (A). In the parallel flow, for example, the blood enters into the packed filter material 4 from the holes for liquid passage 32, and flows in the filter material 4 along the arrow directions shown in Figs. 17 (A) and (B), namely, the blood flows so as to form a parallel flow. Where, as the form of the filter material to be processed, any form may be employed as long as the blood flows as a parallel flow relative to the filter material 4, and as such a structural material, various forms other than that shown in the figure can be employed.

### Examples

Hereinafter, the present invention will be explained in more detail based on examples. Where, as the methods for determination in the examples, the following methods were employed.

### A. Melt viscosity of polymer:

The melt viscosity of polymer was determined by Capilograph 1B produced by Toyo Seiki Corporation. Where, the waiting time of polymer from sample deposition to start of determination was set at ten minutes.

### B. Melting point:

Using DSC-7 produced by Perkin Elmaer Corporation, the peak top temperature indicating a fusion of polymer at 2nd run was determined as the melting point of the polymer. At that time, the heating rate was controlled at 16°C/min., and the amount of sample was set at 10 mg.

### C. Uster nonuniformity of polymer alloy fiber (U%):

Using USTER TESTER produced by Zelbegar Corporation, it was determined at a yarn supplying speed of 200 m/min. and at a normal mode.

### D. Observation of cross section of medical filter material by TEM:

A medical filter material was embedded with an epoxy resin, a very thin piece was cut out in the cross-sectional direction, and the cross section of the medical filter material was observed by a transmission electron microscope (TEM). Further, a metal dyeing was carried out as needed.
TEM: H-7100FA Type produced by Hitachi Co., Ltd.

### E. Number average diameter of nanofibers:

The number average diameter of nanofibers was determined as follows. Namely, A photograph of a cross section of a medical filter material taken by TEM was analyzed by an image analyzing soft (WINROOF) and the diameters of nanofibers were calculated, and a simple average value thereof was determined. The average value was calculated by measuring the diameters of 50 or more nanofibers sampled randomly in an identical cross section as the number of nanofibers, carrying out this measurement at three or more positions, and using totally 150 or more nanofibers.

### F. Calculation of fiber ratio of single fibers with a diameter in a range of more than 500 nm and 1 µm or less:

The fiber ratio of single fibers with a diameter in a range of more than 500 nm and 1µm or less in a medical filter material was determined as follows. Namely, respective diameters of single fibers of nanofibers in the medical filter material are referred to as "di", and the total sum of squares thereof is calculated ((d₁²+d₂²+ · · · +dₙ²) = Σdᵢ²(i=1-n)). Further, respective diameters of nanofibers in a range of more than 500 nm and 1µm or less are referred to as "Di", and the total sum of squares thereof is calculated ((D₁²+D₂²+ · · · +Dₘ²) = ΣDᵢ²(i=1-m)). By calculating the rate of ΣDᵢ² to Σdᵢ², the area ratio of fibers with a diameter in a range of more than 500 nm and 1µm or less relative to all nanofibers, that is, the ratio in terms of weight ratio, can be determined.

### G. SEM observation:

A filter substrate was deposited with platinum, and it was observed by a ultra resolution field emission-type scanning electron microscope.
Ultra resolution field emission-type SEM: UHR-FE-SEM produced by Hitachi Co., Ltd.

### H. Mechanical properties:

10 m of polymer alloy fibers was sampled, the weight thereof was measured at a condition of sample number of 5, and from an average value thereof, the fineness was determined (dtex). Then, the load-elongation curve was determined at a condition indicated in JIS L1013 at measurement conditions of a room temperature (25°C), an initial sample length of 200 mm and a tensile speed of 200 mm/min. Next, the load value at breakage was divided by the initial fineness, it was determined as a strength, and the elongation at breakage was divided by the initial sample length, it was determined as an elongation, and therefrom, strength-elongation curve was determined.

### I. Fiber concentration of nanofibers in secondarily beaten fibers:

Secondarily beaten fibers were metered by 1g, it was evaporated and dried, and the concentration was determined from the weight of the residue.

### J. Fiber concentration in nanofiber water dispersion:

Nanofiber water dispersion was metered by 10g, it was evaporated and dried, and the concentration was determined from the weight of the residue.

### K. Weight:

The weight of a medical filter material was measured by JIS L1096 8.4.2 (1999).

### L. Apparent density:

The above-described weight of a filter material and a thickness thereof were measured, and an average value of the apparent densities obtained therefrom was defined as an apparent density. Where, for the measurement of the thickness, a dial thickness gauge "Peacock H" produced by Ozaki Seisakusyo Corporation was used, ten points of the sample were measured, and the average value was used.

### M. Evaluation due to electrophoresis/one dimensional expansion method (SDS-PAGE method):

10 mg of filter substrate was dipped in 200µL of 1% SDS (sodium dodecyl sulfate) solution, and after ultrasonic treatment was carried out for one hour, extraction of protein adhered to the filter substrate was carried out by still standing at 4°C for one night. 20µL of extracted solution taken out was dried by an centrifugal evaporator, it was added with sample buffer (58 mM Tris/HCl (pH: 6.8), 1.8% SDS, 5% glycerol, 0.05% bromphenol blue) and it was dissolved again, and heat treatment was carried out at 100°C for 5 minutes. This was applied to SDS-PAGE gel (polyacrylamide gel: 4-20% gradient gel, 1 mm thickness, produced by TEFCO Corporation), and it was served to electrophoresis at 18 mA for 90 minutes (buffer of electrophoresis: 25 mM Tris, 0.19M glycine, 0.1% SDS). Further, the gel after electrophoresis was dyed at silver (silver dyeing kit, produced by Wako Junyaku Kogyo Corporation) and protein was detected.

### Example 1:

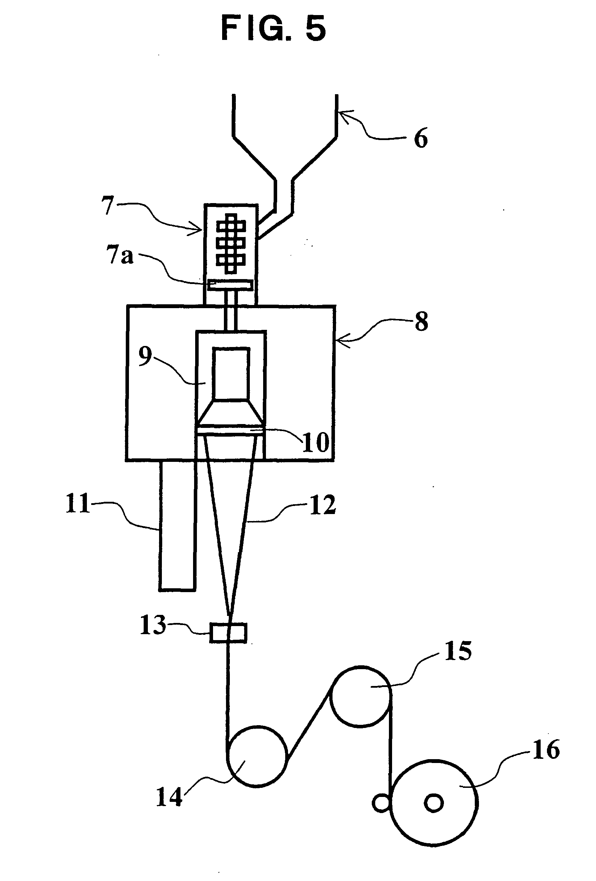

N6 [nylon 6] (20% by weight) with a melt viscosity of 53 Pa · s (at 262°C, shear speed: 121.6 sec⁻¹) and a melting point of 220 °C and PET [polyethylene terephthalate] copolymerized with 8 mol% isophthalic acid and 4 mol% bisphenol A with a melt viscosity of 310 Pa · s (at 262°C, shear speed: 121.6 sec⁻¹) and a melting point of 225°C (80% by weight) were kneaded at 260°C by a twin-screw extruding kneader to prepare polymer alloy chips. Where, the melt viscosity at 262°C and 121.6 sec⁻¹ of this copolymerized PET obtained was 180 Pa · s. The kneading condition at that time was as follows. As to polymer supply, N6 and the copolymerized PET were metered separately, and supplied to the kneader separately. A screw with a diameter of 37 mm, an effective length of 1670 mm and L/D (ratio of screw part length/diameter) of 45.1 was used. A model diagram of a melt spinning apparatus used for the melt spinning is shown in Fig. 5. In Fig. 5, label 6 indicates a hopper, label 7 indicates a chip supplying part, label 7a indicates a melting part thereof, label 8 indicates a spin block, label 9 indicates a spinning pack, label 10 indicates a die, label 11 indicates a chimney, label 12 indicates a yarn melt spun, label 13 indicates a collecting and oiling guide, label 14 indicates a first godet roller, label 15 indicates a second godet roller, and label 16 indicates a wound yarn, respectively.

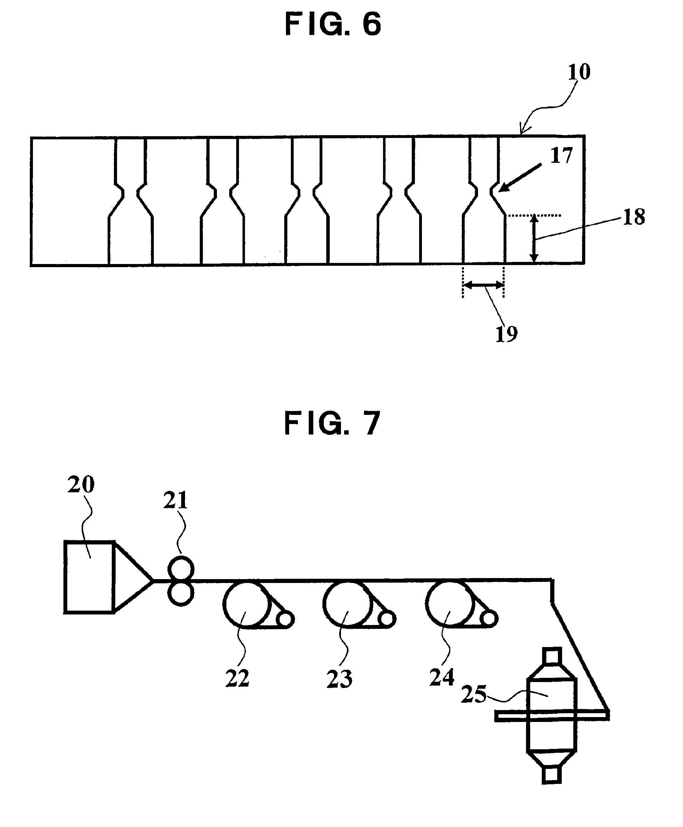

The above-described polymer alloy chips were molten at 275°C at melting part 7a, and introduced into spin block 8. Then, after the polymer alloy molten material was filtered by a metal medical filter material with a critical filtration diameter of 15µm, it was melt spun from die 10 the die surface temperature of which was controlled at 262°C. At that time, as the die 10, as shown in Fig. 6, a die with a metering zone 17 at an upper part of the discharge hole having a diameter of 0.3 mm, and with a diameter of discharge hole 19 of 0.7 mm and a length of discharge hole 18 of 1.75 mm, was used. The discharge amount per a single hole at that time was controlled at 2.9 g/min. Further, the distance from the lower surface of the die to a cooling start point (an upper part of chimney 11) was 9 cm. As shown in Fig. 5, the discharged yarn was cooled and solidified over 1m at the chimney part by cooling air controlled at 20°C, and after the yarn was supplied with oil at collecting and oiling guide 13 installed below by 1.8m from die 10, it was wound at 900 m/min. through first godet roller 14 and second godet roller 15 non-heated.

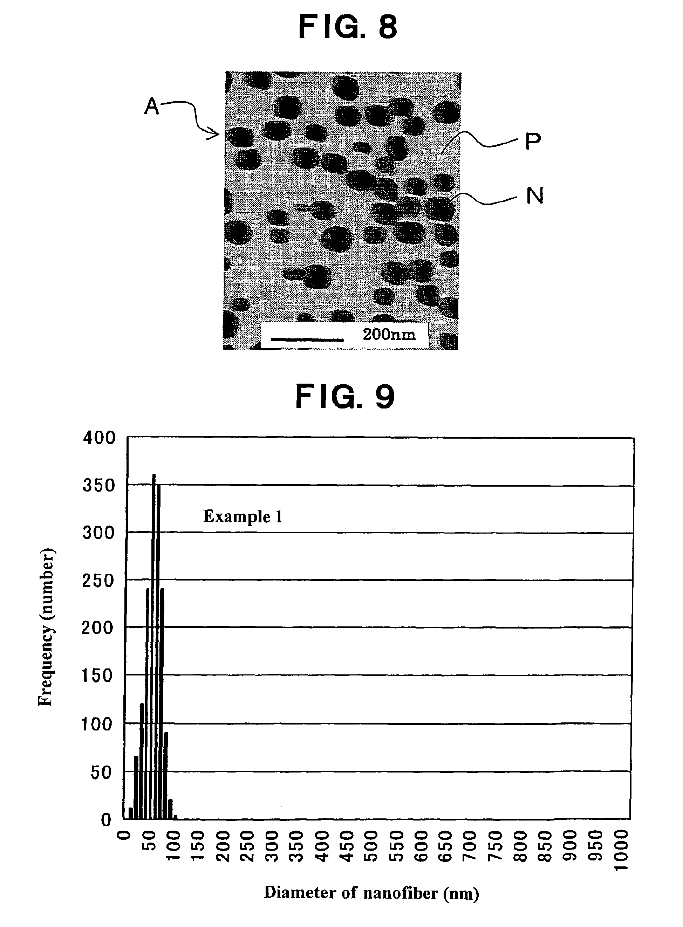

Then, using a drawing apparatus the schematic model diagram of which was shown in Fig. 7, it was drawn and heat treated through a first hot roller 22 controlled at a temperature of 90°C and a second hot roller 23 controlled at a temperature of 130°C. At that time, the draw ratio between the first hot roller 22 and the second hot roller 23 was controlled at 3.2 times. In Fig. 7, label 20 indicates a non-drawn yarn, label 21 indicates a feed roller, label 24 indicates a third roller with a room temperature, and label 25 indicates a wound drawn yarn, respectively. The obtained polymer alloy fibers exhibited excellent properties of 120 dtex, 12 filaments, a strength of 4.0 cN/dtex, an elongation of 35% and U%=1.7%. Further, when the cross section of the obtained polymer alloy fibers was observed by TEM, as shown in Fig. 8, polymer alloy fibers (label A) were obtained, wherein a sea/island structure with a sea component of the copolymerized PET (light color part: label P) and an island component of N6 (heavy color part: label N) was provided, and the number average diameter of island N6 was 53 nm, and which was a precursor of nanofibers in which N6 was dispersed very finely.

By dipping the polymer alloy fibers in 5% sodium hydroxide aqueous solution with a temperature of 95°C for one hour, 99% or more of polyester component in the polymer alloy fibers was removed by hydrolysis, and after neutralized with acetic acid, they were washed with water and dried, they were cut at a length of 2 mm by a guillotine cutter to obtain cut fibers of N6 nanofibers. When the polymer alloy fibers were knitted at a circular knitting separately, they were made to nanofibers in a manner similar to the above-described manner, they were pulled out from the circular knitting and a strength of the yarn was measured, a sufficient strength of 2 cN/dtex was exhibited. Further, an oriented and crystallized condition was confirmed by determination of X-ray diffraction.

23L of water and 30g of the above-described cut fibers were charged into a TAPPI standard Niagara test beater (produced by Toyo Seiki Seisakusyo Corporation), primarily beaten for 5 minutes, and thereafter, excessive water was removed and fibers were recovered. The fiber concentration after this primary beating was 10% by weight. The primarily beaten fibers were charged into an automatic PFI mil (produced by Kumagaya Riki Kogyo Corporation), and secondarily beaten at a rotational speed of 1500 rpm and a clearance of 0.2 mm for 6 minutes. The fiber concentration after the secondary beating was 10% by weight.

250g of fibers containing water were were charged into an automatic PFI mil (produced by Kumagaya Riki Kogyo Corporation) as they were, and beaten at a rotational speed of 1500 rpm and a clearance of 0.2 mm for 6 minutes. 4.2g of the beaten fibers, 0.5g of Charol AN-103P (produced by Daiichi Kogyo Seiyaku Corporation) as a dispersant and 500g of water were charged into a fiber mixer MX-X103 (produced by Matsushita Denki Sangyo Corporation), and stirred for 5 minutes, and a water dispersion of N6 nanofibers was obtained. The fiber concentration in the water dispersion was 0.08% by weight. 500g of the obtained water dispersion of N6 nanofibers and 20L were charged into a rectangular sheet machine, after made as a paper on a No. 2 qualification filter paper (produced by Toyo Roshi Corporation), it was dried as it was at 110°C using a high-temperature rotational type drier (produced by Kumagaya Riki Kogyo Corporation), and by removing the fiber sheet part from the filter paper, a medical filter material made of only nanofibers was obtained.

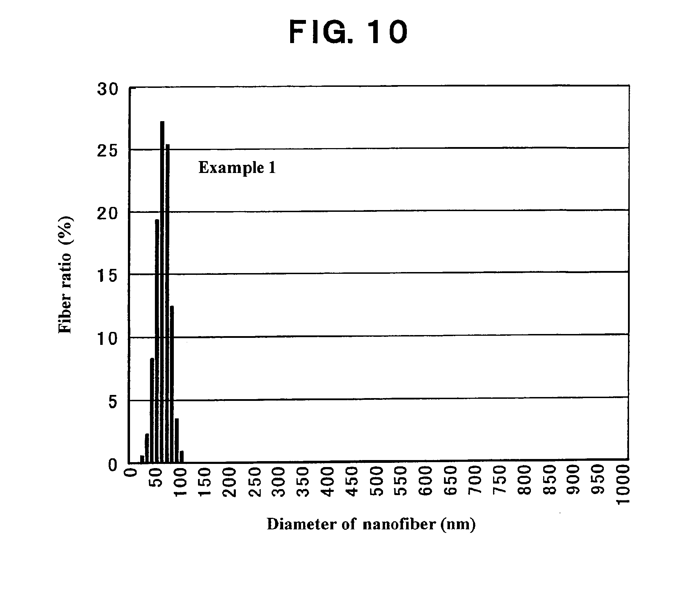

When the surface of this medical filter material was observed by SEM, as shown in Fig. 2, a structure where the nanofibers were dispersed like a net was observed. The result of analyzing the diameters of the nanofibers from TEM photograph was shown as histograms in Figs. 9 and 10, the number average diameter of the nanofibers was 56 nm which was a fineness that had not been present in the conventional technology, the fiber ratio of single fibers with a diameter in a range of more than 500 nm and 1µm or less was 0%, the fiber ratio of single fibers with a diameter more than 200 nm was 0%, and the fiber ratio of single fibers with a diameter more than 100 nm was 0%. This result is shown in Table 1.

Further, the weight of the medical filter material was 8 g/m², and the apparent density thereof was 0.27 g/cm³.

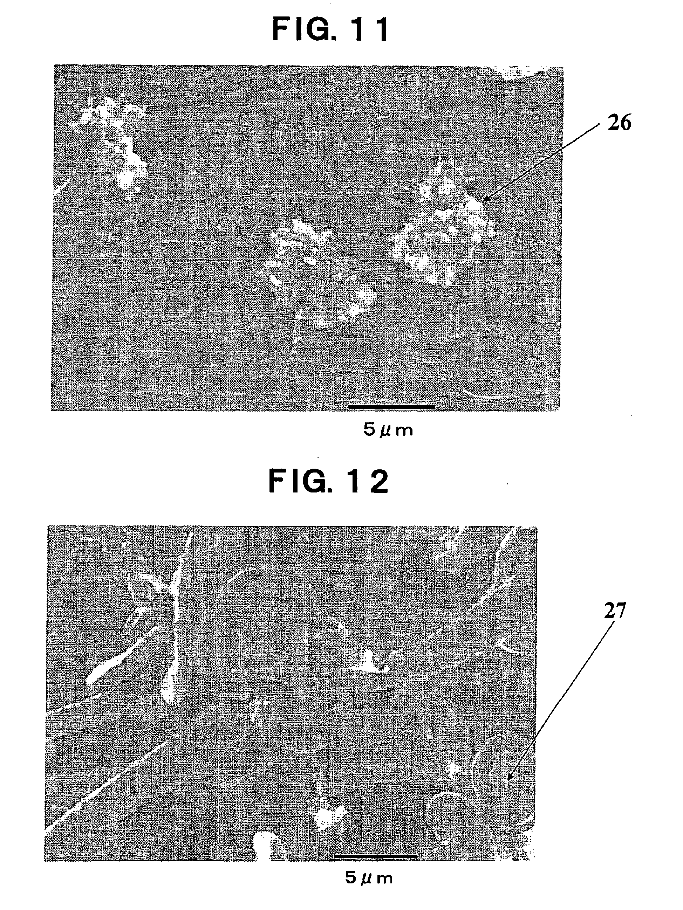

This filter material was adhered to a wall surface of Eppendorph tube with a double coated tape, after it was dipped in a human blood suppressed in coagulation by heparin at 38 °C for one hour, it was washed sufficiently in physiological brine. After the filter material after washing was dipped in 0.1% glutaric aldehyde solution for one night to fix the hemocyte surface, the filter material was freeze dried. When the surface of the filter material after freeze drying was observed by SEM, many leukocytes were adsorbed on the surface of the filter material. The result of the observation of the surface of the filter material after evaluation of hemocyte adsorption in Example 1 is shown in Fig. 11. Label 26 in Fig. 11 is leukocyte.

### Comparative Examples 1 and 2:

As a filter material, in Comparative Example 1, a very fine N6 fiber felt with a fiber diameter of 4µm, a weight of 270 g/m² and an apparent density of 0.5 g/cm³ was used, and in Comparative Example 2, a very fine PET fiber felt with a fiber diameter of 5 µm, a weight of 270 g/m² and an apparent density of 0.4 g/cm³ was used. As the result of evaluating the hemadsorption of these felts by dipping them in human blood by the same operation as that in Example 1, Comparative Examples 1 and 2 were different from Example 1, the adsorption of leukocytes onto the surface of the filter materials were not present, and most of them were erythrocytes. The result of the observation of the surface of the filter material after evaluation of hemocyte adsorption by SEM in Comparative Example 1 is shown in Fig. 12. Label 27 in Fig. 12 indicates erythrocyte.

### Example 2 and Comparative Example 3:

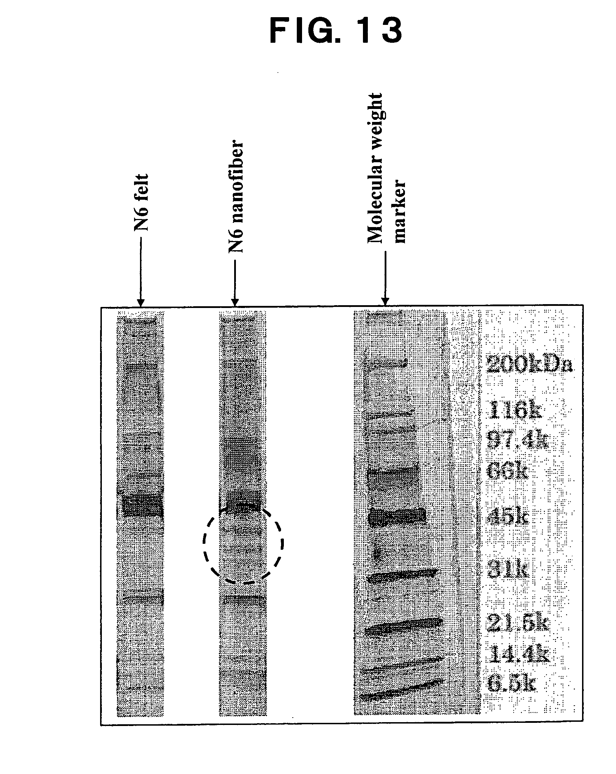

In Example 2, the filter material prepared in Example 1 was used, and in Comparative Example 3, the filter material used in Comparative Example 1 was used, respectively. After the respective fiber substrates were dipped in human blood plasma at 38°C for one hour, they were washed sufficiently in physiological brine. After the fiber substrates after washing were freeze dried, the components adsorbed were extracted from the fiber substrates by SDS. When the solutions after extraction were analyzed with respect to the molecular weight of adsorbed protein by electrophoresis/one dimensional expansion method (SDS-PAGE method), as shown in Fig. 13, although an adsorption was not observed in the N6 felt of Comparative Example 3, it was understood that, in the filter material of N6 nanofibers in Example 2, protein with a molecular weight of 31,000 to 45,000 was significantly adsorbed.

### Example 3:

The filter material obtained in Example 1 was packed into a cylindrical PP (polypropylene) vessel with a diameter of 4.7 cm and a length of 17 cm at a lattice form, and an extracorporeal circulation column was made so as to form a body fluid flow parallel to the filter substrate. The schematic diagram of the lattice form was shown in Fig. 3 (B). When a bovine blood was passed through this column at a flow rate of 2 mL/min. for 90 minutes, an extracorporeal circulation column having a sufficient liquid passage property without clogging could be obtained.

### Example 4:

A PP (polypropylene) nonwoven fabric with a weight of 240 g/m² was prepared by applying carding and wrapping to PP raw stock with a single fiber fineness of 1.9 dtex and further carrying out needle punching at a punch density of 500/cm². 5g of the water dispersion of N6 nanofibers prepared in Example 1 and 20L of water were charged into a rectangular sheet machine, after made as a paper on the PP nonwoven fabric, it was dried as it is at 110°C using a high-temperature rotational type drier (produced by Kumagaya Riki Kogyo Corporation), thereby obtaining a medical filter material in which N6 nanofibers were dispersed on the PP nonwoven fabric in a net-like form. The weight of this medical filter material was 240 g/m², and the apparent density thereof was 0.02 g/m³.

Only N6 nanofibers were taken out from the obtained filter material, and as the result of analyzing in the same manner as that in Example 1, the number average diameter of the nanofibers was 56 nm which was a fineness that had not been present in the conventional technology, and the fiber ratio of single fibers with a diameter more than 100 nm was 0%. This result is shown in Table 1.

This filter material was cut at a circle with a diameter of 4.7 cm, ten cut pieces were stacked and they were charged into a cylindrical PP vessel in the same manner as that of Example 3, to make a column so as to form a body fluid flow perpendicular to the filter material. When a bovine blood was passed through this column similarly to in Example 3, an extracorporeal circulation column having a sufficient liquid passage property without clogging could be obtained.

### Example 5:

Using a polystyrene (co-PS) copolymerized with 22% of PBT (polybutylene terephthalate) with a melt viscosity of 120 Pa · s (at 262°C, 121.6 sec⁻¹) and a melting point of 225°C and 2-ethylhexyl acrylate, melt kneading was carried out similarly to in Example 1 at a content of PBT of 23% by weight and a kneading temperature of 240°C to obtain polymer alloy chips. They were melt spun similarly to in Example 1 at a melting temperature of 260 °C, a spinning temperature of 260°C (surface temperature of a die: 245°C), a discharge amount of a single hole of 1.0 g/min. and a spinning speed of 1200 m/min. The non-drawn yarn obtained was drawn and heat treated at a drawing temperature of 100°C, a draw ratio of 2.49 times and a heat set temperature of 115°C similarly to in Example 1. The obtained drawn yarn was 161 dtex, 36 filaments, and the strength thereof was 1.4 cN/dtex, the elongation was 33%, and U% was 2.0%.

When the cross section of the obtained polymer alloy fibers was observed by TEM, a sea/island structure with the sea of co-PS and the island of PBT was exhibited, the number average diameter of PBT was 60 nm, and polymer alloy fibers in which PBT was uniformly dispersed at a nano size were obtained.

After 99% or more of co-PS, which was the sea component, was dissolved out by dipping the polymer alloy fibers in trichlene, they were dried, and they were cut at a length of 2 mm by a guillotine cutter to obtain cut fibers of PBT nanofibers. When the polymer alloy fibers were knitted at a circular knitting separately, they were made to nanofibers in a manner similar to the above-described manner, they were pulled out from the circular knitting and a strength of the yarn was measured, a sufficient strength of 1.5 cN/dtex was exhibited. Further, an oriented and crystallized condition was confirmed by determination of X-ray diffraction.

Secondarily beaten fibers were obtained from the cut fibers in a manner similar to in Example 1. The fiber concentration after the secondary beating was 20% by weight. 2.1 g of the fibers after secondary beating, 0.5g of "Noigen" EA-87 (produced by Daiichi Kogyo Seiyaku Corporation) as a dispersant and 500g of water were charged, they were stirred for 5 minutes to obtain a water dispersion of PBT nanofibers. The fiber concentration in the water dispersion was 0.08% by weight.

5g of the water dispersion of PBT nanofibers obtained and 20L of water were charged into a rectangular sheet machine, after made as a paper on the PP nonwoven fabric prepared in Example 4, it was dried as it was at 110°C using a high-temperature rotational type drier (produced by Kumagaya Riki Kogyo Corporation), thereby obtaining a medical filter material in which PBT nanofibers were dispersed on the PP nonwoven fabric in a net-like form. The weight of this filter material was 240 g/m², and the apparent density thereof was 0.02 g/m³.

Only PBT nanofibers were taken out from the obtained filter material, and as the result of analyzing in the same manner as that in Example 1, the number average diameter of the nanofibers was 80 nm which was a fineness that had not been present in the conventional technology, the fiber ratio of single fibers with a diameter more than 200 nm was 0%, and the fiber ratio of single fibers with a diameter more than 100 nm was 1% or less. This result is shown in Table 1.

This filter material was charged into a cylindrical PP vessel in the same manner as that in Example 3, to make a column so as to form a body fluid flow perpendicular to the filter material. When a bovine blood was passed through this column similarly to in Example 3, an extracorporeal circulation column having a sufficient liquid passage property without clogging could be obtained.

### Example 6:

21% by weight of PP with a melt viscosity of 250 Pa · s (at 220°C, 121.6 sec⁻¹) and a melting point of 162°C and 79% by weight of poly-L-lactic acid (optical purity: 99.5% or more) with a weight average molecular weight of 120,000, a melt viscosity of 30 Pa s (at 240°C, shear speed: 2432 sec⁻¹) and a melting point of 170°C were melt kneaded at a kneading temperature of 220°C similarly to that in Example 1 to prepare polymer alloy chips. They were melt spun similarly to in Example 1 at a melting temperature of 220°C, a spinning temperature of 220°C (surface temperature of a die: 205°C), a discharge amount of a single hole of 2.0 g/min. and a spinning speed of 1200 m/min. The non-drawn yarn obtained was drawn and heat treated at a drawing temperature of 90°C, a draw ratio of 2.0 times and a heat set temperature of 130°C similarly to in Example 1. The obtained drawn yarn was 101 dtex, 12 filaments, and the strength thereof was 2.0 cN/dtex and the elongation was 47%.

When the cross section of the obtained polymer alloy fibers was observed by TEM, a sea/island structure with the sea of poly-L-lactic acid and the island of PP was exhibited, the number average diameter of PP was 150 nm, and polymer alloy fibers in which PP was uniformly dispersed at a nano size were obtained.

By dipping the obtained polymer alloy fibers in 5% sodium hydroxide aqueous solution with a temperature of 98°C for one hour, 99% or more of poly-L-lactic component in the polymer alloy fibers was removed by hydrolysis, and after neutralized with acetic acid, they were washed with water and dried, they were cut at a length of 2 mm by a guillotine cutter to obtain cut fibers of PP nanofibers. Secondary beaten fibers were obtained from the cut fibers similarly to in Example 1. The fiber concentration after secondary beating was 25% by weight. When the polymer alloy fibers were knitted at a circular knitting separately, they were made to nanofibers in a manner similar to the above-described manner, they were pulled out from the circular knitting and a strength of the yarn was measured, a sufficient strength of 1.5 cN/dtex was exhibited. Further, an oriented and crystallized condition was confirmed by determination of X-ray diffraction.

1.7g of the fibers after secondary beating, 0.5g of "Noigen" EA-87 (produced by Daiichi Kogyo Seiyaku Corporation) as a dispersant and 500g of water were charged, they were stirred for 5 minutes to obtain a water dispersion of PP nanofibers. The fiber concentration in the water dispersion was 0.08% by weight.

5g of the water dispersion of PP nanofibers obtained and 20L of water were charged into a rectangular sheet machine, after made as a paper on the PP nonwoven fabric prepared in Example 4, it was dried as it was at 110°C using a high-temperature rotational type drier (produced by Kumagaya Riki Kogyo Corporation), thereby obtaining a medical filter material in which PP nanofibers were dispersed on the PP nonwoven fabric in a net-like form. The weight of the medical filter material obtained was 240 g/m², and the apparent density thereof was 0.02 g/m³.

Only PP nanofibers were taken out from the obtained filter material, and as the result of analyzing in the same manner as that in Example 1, the number average diameter of the nanofibers was 160 nm which was a fineness that had not been present in the conventional technology, the fiber ratio of single fibers with a diameter of more than 500 nm and 1µm or less was 0%, and the fiber ratio of single fibers with a diameter more than 200 nm was 1.8%. This result is shown in Table 1.

This filter material was charged into a cylindrical PP vessel in the same manner as that in Example 4, to make a column so as to form a body fluid flow perpendicular to the filter material. When a bovine blood was passed through this column similarly to in Example 3, an extracorporeal circulation column having a sufficient liquid passage property without clogging could be obtained.

### Example 7:

Using 40% by weight of N6 with a melt viscosity of 500 Pa · s (at 262°C, shear speed: 121.6 sec⁻¹) and a melting point of 220°C, melt spinning was carried out similarly to in Example 1 to obtain polymer alloy fibers. The obtained polymer alloy fibers were 126 dtex, 36 filaments, and they exhibited excellent properties of a strength of 4.2 cN/dtex, an elongation of 38%, and U%=1.8%.

Further, when the cross section of the obtained polymer alloy fibers was observed by TEM, a sea/island structure with the sea of copolymerized PET and the island of N6 was exhibited similarly to in Example 1, the number average diameter of the island N6 was 80 nm, and polymer alloy fibers in which N6 was uniformly dispersed very finely were obtained.

By dipping the obtained polymer alloy fibers in 5% sodium hydroxide aqueous solution with a temperature of 98°C for one hour, 99% or more of polyester component in the polymer alloy fibers was removed by hydrolysis, and after neutralized with acetic acid, they were washed with water and dried, they were cut at a length of 2 mm by a guillotine cutter to obtain cut fibers of N6 nanofibers. Secondary beaten fibers were obtained from the cut fibers similarly to in Example 1. The fiber concentration after secondary beating was 12% by weight. When the polymer alloy fibers were knitted at a circular knitting separately, they were made to nanofibers in a manner similar to the above-described manner, they were pulled out from the circular knitting and a strength of the yarn was measured, a sufficient strength of 2 cN/dtex was exhibited. Further, an oriented and crystallized condition was confirmed by determination of X-ray diffraction.

4.0g of the fibers after secondary beating, 0.5g of the same dispersant as that in Example 1 and 500g of water were charged, they were stirred for 5 minutes to obtain a water dispersion of N6 nanofibers. The fiber concentration in the water dispersion was 0.1% by weight.

5g of the water dispersion of N6 nanofibers obtained and 20L of water were charged into a rectangular sheet machine, after made as a paper on the PP nonwoven fabric prepared in Example 4, it was dried as it was at 110°C using a high-temperature rotational type drier (produced by Kumagaya Riki Kogyo Corporation), thereby obtaining a medical filter material in which N6 nanofibers were dispersed on the PP nonwoven fabric in a net-like form. The weight of the medical filter material obtained was 240 g/m², and the apparent density thereof was 0.02 g/m³.

Only the nanofibers were taken out from the obtained filter material, and as the result of analyzing in the same manner as that in Example 1, the number average diameter of the nanofibers was 84 nm which was a fineness that had not been present in the conventional technology, the fiber ratio of single fibers with a diameter more than 200 nm was 0%, and the fiber ratio of single fibers with a diameter more than 100 nm was 2.2%. This result is shown in Table 1.

This filter material was charged into a cylindrical PP vessel in the same manner as that in Example 3, to make a column so as to form a body fluid flow perpendicular to the filter material. When a bovine blood was passed through this column similarly to in Example 3, an extracorporeal circulation column having a sufficient liquid passage property without clogging could be obtained.

### Example 8:

Using N6 used in Example 1 and the same poly-L-lactic acid as that used in Example 6 and controlling the content of N6 at 20% by weight, they were melt kneaded at a kneading temperature of 220°C similarly to that in Example 1 to prepare polymer alloy chips. They were melt spun similarly to in Example 1 at a melting temperature of 230°C, a spinning temperature of 230°C (surface temperature of a die: 215°C) and a spinning speed of 3200 m/min. to obtain a non-drawn yarn. The non-drawn yarn obtained was drawn and heat treated at a drawing temperature of 90°C, a draw ratio of 1.5 times and a heat set temperature of 130°C similarly to in Example 1 to obtain polymer alloy fibers. The obtained polymer alloy fibers were 70 dtex, 36 filaments, and the strength thereof was 3.4 cN/dtex, the elongation was 38%, and U% was 0.7%.

When the cross section of the obtained polymer alloy fibers was observed by TEM, a sea/island structure with the sea of poly-L-lactic acid and the island of N6 was exhibited, the number average diameter of N6, which was the island component, was 55 nm, and polymer alloy fibers in which N6 was uniformly dispersed at a nano size were obtained.

By dipping the obtained polymer alloy fibers in 5% sodium hydroxide aqueous solution with a temperature of 98°C for one hour, 99% or more of poly-L-lactic component in the polymer alloy fibers was removed by hydrolysis, and after neutralized with acetic acid, they were washed with water and dried, they were cut at a length of 2 mm by a guillotine cutter to obtain cut fibers of N6 nanofibers. Secondary beaten fibers were obtained from the cut fibers similarly to in Example 1. The fiber concentration after secondary beating was 10% by weight. When the polymer alloy fibers were knitted at a circular knitting separately, they were made to nanofibers in a manner similar to the above-described manner, they were pulled out from the circular knitting and a strength of the yarn was measured, a sufficient strength of 2 cN/dtex was exhibited. Further, an oriented and crystallized condition was confirmed by determination of X-ray diffraction.

4.5g of the fibers after secondary beating, 0.5g of the same dispersant as that of Example 1 and 500g of water were charged, they were stirred for 5 minutes to obtain a water dispersion of N6 nanofibers. The fiber concentration in the water dispersion was 0.09% by weight.

5g of the water dispersion of N6 nanofibers obtained and 20L of water were charged into a rectangular sheet machine, after made as a paper on the PP nonwoven fabric prepared in Example 4, it was dried as it was at 110°C using a high-temperature rotational type drier (produced by Kumagaya Riki Kogyo Corporation), thereby obtaining a medical filter material in which N6 nanofibers were dispersed on the PP nonwoven fabric in a net-like form. The weight of the medical filter material obtained was 240 g/m², and the apparent density thereof was 0.02 g/m³.

Only the nanofibers were taken out from this filter material, and as the result of analyzing in the same manner as that in Example 1, the number average diameter of the nanofibers was 56 nm, and the fiber ratio of single fibers with a diameter more than 100 nm was 0%. This result is shown in Table 1.

This filter material was charged into a cylindrical PP vessel in the same manner as that in Example 4, to make a column so as to form a body fluid flow perpendicular to the filter material. When a bovine blood was passed through this column similarly to in Example 3, an extracorporeal circulation column having a sufficient liquid passage property without clogging could be obtained.

### Examples 9 to 13:

The filter substrate of Example 4 was used in Example 9, the filter substrate of Example 5 was used in Example 10, the filter substrate of Example 6 was used in Example 11, the filter substrate of Example 7 was used in Example 12, and the filter substrate of Example 8 was used in Example 13, respectively. Each filter substrate was packed into a thin-type vessel shown in Fig. 4 having a blood introduction port at its upper part and a blood discharge port at its lower part to make a blood filter. When a bovine blood was passed through the respective blood filters at a flow rate of 2 mL/min. for 20 minutes, blood filters having a sufficient liquid passage property without clogging could be obtained.

### Example 14:

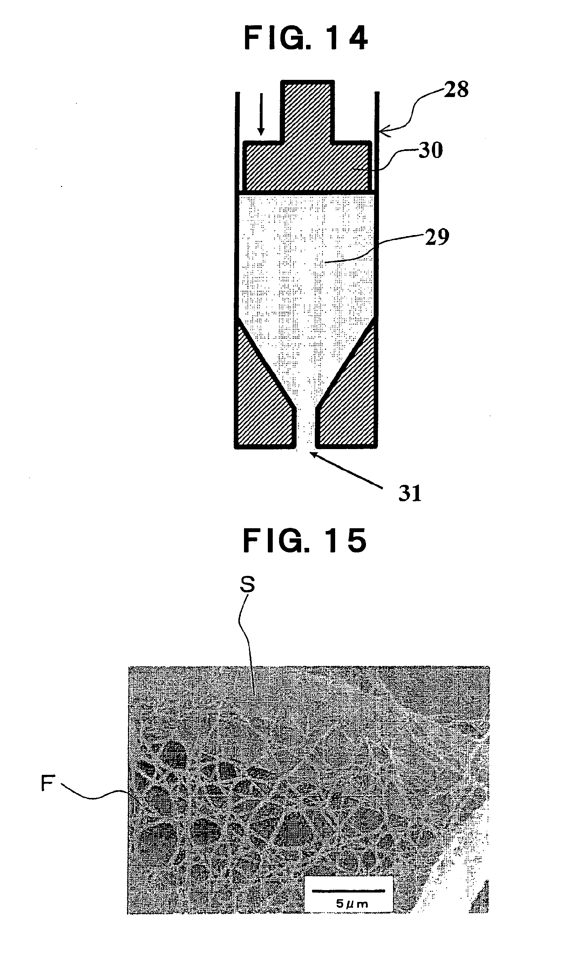

After the polymer alloy fibers prepared in Example 1 were turned to bundles using a hank, they were packed into a silicone heat-shrinkage tube ("Nishitube" NST, produced by Nishinihon Densen Corporation). Thereafter, it was heat treated under a pressure-reduced condition at 180°C for 2 hours to prepare a rod made of the polymer alloy fibers having a diameter of 3 cm and a length of 20 cm. This was deposited into the extruder 28 shown in Fig. 14, the rod 29 made of the polymer alloy fibers was extruded by the piston 31 from the discharge port 31 with a diameter of 1 cm at 235°C to obtain melt-drawn fibers. When the cross section thereof was observed by TEM, a sea/island structure with the sea component of copolymerized PET and the island component of N6 was exhibited, the number average diameter of the island N6 was 20 nm, and polymer alloy fibers, in which N6 was dispersed very finely and which were a precursor of nanofibers, were obtained.

A water dispersion was obtained from the polymer alloy fibers obtained similarly to in Example 1. The fiber concentration in the water dispersion was 0.08% by weight. The water dispersion obtained was made as a paper on the PP nonwoven fabric similarly to in Example 4, and a medical filter material, in which N6 nanofibers were dispersed on the PP nonwoven fabric in a net-like form, was obtained. The weight of the filter material obtained was 240 g/m², and the apparent density thereof was 0.02 g/m³. Only the nanofibers were taken out from the obtained filter material, and as the result of analyzing in the same manner as that in Example 1, the number average diameter of the nanofibers was 25 nm which was a fineness that had not been present in the conventional technology, and the fiber ratio of single fibers with a diameter more than 100 nm was 0%.

This filter material was packed into a thin-type vessel having a blood introduction port at its upper part and a blood discharge port at its lower part similarly to in Example 9 to make a blood filter. When a bovine blood was passed through this blood filter similarly to in Example 9, although the blood filter had a sufficient liquid passage property without clogging, there was a case where a very small amount of suspended substances, which seemed to be agglomerated substances of fine fiber waste to the bovine blood after passing through the filter material, were mixed. This is considered that, because the nanofibers were too thin, a part of the nanofibers were cut when the bovine blood was passed.

### Example 15:

0.5g of the water dispersion of N6 nanofibers obtained in Example 1 and 500g of water were charged into a sprayer and the mixture was sprayed several times onto a PET nonwoven fabric with a fiber diameter of 7µm, a weight of 150 g/m² and an apparent density of 0.16 g/cm³, to obtain a medical filter material in which N6 nanofibers were dispersed at a net-like form. The result of observing the surface of the filter material by SEM was shown in Fig. 15. In Fig. 15, label F indicates nanofibers, and label S indicates the fibers of the substrate. The weight of the obtained medical filter material was 150 g/m² and the apparent density thereof was 0.16 g/cm³. Only the N6 nanofibers were taken out from the obtained filter material, and as the result of analyzing in the same manner as that in Example 1, the number average diameter of the nanofibers was 56 nm which was a fineness that had not been present in the conventional technology, and the fiber ratio of single fibers with a diameter more than 100 nm was 0%.

The obtained filter material was packed into a thin-type vessel similarly to in Example 9 to make a blood filter. When a bovine blood was passed through this blood filter similarly to in Example 9, a blood filter having a sufficient liquid passage property without clogging was obtained.

The conditions and the results in Examples 1 to 15 are shown in Table 1 and Table 2.

**[Table 1]**

| | Nanofiber | | Fiber ratio of single fibers of nanofibers | | |
|---|---|---|---|---|---|
| | Kind of polymer | Number average diameter (nm) | Diameter range of more than 500nm and 1µm or less | Diameter range of more than 200nm and 1µm or less | Diameter range of more than 100nm and 1µm or less |
| Example 1 | N6 | 5.6 | 0% | 0% | 0% |
| Example 2 | N6 | 56 | 0% | 0% | 0% |
| Example 3 | N6 | 56 | 0% | 0% | 0% |
| Example 4 | N6 | 56 | 0% | 0% | 0% |
| Example 5 | PBT | 50 | 0% | 0% | 0% |
| Example 6 | PP | 150 | 0% | 1.8% | - |
| Example 7 | N6 | 84 | 0% | 0% | 2.2% |
| Example 8 | N6 | 56 | 0% | 0% | 0% |
| Example 9 | N6 | 56 | 0% | 0% | 0% |
| Example 10 | PBT | 50 | 0% | 0% | 0% |
| Example 11 | PP | 150 | 0% | 1.8% | - |
| Example 12 | N6 | 84 | 0% | 0% | 2.2% |
| Example 13 | N6 | 56 | 0% | 0% | 0% |
| Example 14 | N6 | 25 | 0% | 0% | 0% |
| Example 15 | N6 | 56 | 0% | 0% | 0% |

**[Table 2]**

| | Weight (g/m²) | Apparent density (g/cm³) |
|---|---|---|
| Example 1 | 8 | 0.27 |
| Example 2 | 8 | 0.27 |
| Example 3 | 240 | 0.02 |
| Example 4 | 240 | 0.02 |
| Example 5 | 240 | 0.02 |
| Example 6 | 240 | 0.02 |
| Example 7 | 240 | 0.02 |
| Example 8 | 240 | 0.02 |
| Example 9 | 240 | 0.02 |
| Example 10 | 240 | 0.02 |
| Example 11 | 240 | 0.02 |
| Example 12 | 240 | 0.02 |
| Example 13 | 240 | 0.02 |
| Example 14 | 240 | 0.02 |
| Example 15 | 150 | 0.16 |

### Industrial Applications of the Invention

The medical filter material according to the present invention is excellent in hemadsorption performance and protein adsorption performance, and therefore, it can be applied to high-performance medical equipment and material using it, in particular, to a medical product for medical treatment such as extracorporeal circulation column or a blood filter.

## Claims

1. A medical filter material **characterized by** comprising a dispersion of nanofibers of thermoplastic polymer having a number average diameter of 1 nm or more and 500 nm or less wherein a ratio of single fibers with a diameter of more than 500 nm and 1µm or less is 3% or less in terms of weight ratio.

2. The medical filter material according to claim 1, wherein at least a part of said dispersion of nanofibers forms a net structure.

3. The medical filter material according to claim 1 or 2, wherein said medical filter material is formed by only said dispersion of nanofibers.

4. The medical filter material according to any of claims 1 to 3, wherein said medical filter material is formed by said dispersion of nanofibers and a substrate of fibers having a number average diameter of more than 1µm and 100µm or less.

5. The medical filter material according to any of claims 1 to 4, wherein said number average diameter of nanofibers is 30 nm or more and 500 nm or less.

6. The medical filter material according to any of claims 1 to 5, wherein a unit weight of said medical filter material is in a range of 1 to 500 g/m².

7. The medical filter material according to any of claims 1 to 6, wherein an apparent density of said medical filter material is in a range of 0.01 1 to 1.0 g/cm³.

8. An extracorporeal circulation column packed with a medical filter material according to any of claims 1 to 7.

9. The extracorporeal circulation column according to claim 8, wherein said medical filter material is packed so as to form a liquid flow perpendicular to said medical filter material.

10. The extracorporeal circulation column according to claim 8, wherein said medical filter material is packed so as to form a liquid flow parallel to said medical filter material.

11. A blood filter packed with a medical filter material according to any of claims 1 to 7.

12. The blood filter according to claim 11, wherein said medical filter material is packed so as to form a liquid flow perpendicular to said medical filter material.

13. The blood filter according to claim 11, wherein said medical filter material is packed so as to form a liquid flow parallel to said medical filter material.
